# EUROPEAN PATENT SPECIFICATION

(11) **EP 4 210 555 B1**
(45) Date of publication and mention of the grant of the patent: **01.04.2026**
(21) Application number: 21866150.2
(22) Date of filing: 10.09.2021
(51) Int. Cl.: A61B 3/10, A61B 5/01, A61B 5/00, A61F 9/008, A61B 5/398

(54) **AN ARRANGEMENT FOR OBTAINING ERG SIGNALS DURING RETINAL HEATING AND CONTROLLING RETINAL HEATING BASED THEREON**
ANORDNUNG ZUR GEWINNUNG VON ERG-SIGNALEN WÄHREND DER RETINALEN ERWÄRMUNG UND STEUERUNG DER RETINALEN ERWÄRMUNG AUF DER BASIS DAVON
AGENCEMENT PERMETTANT D'OBTENIR DES SIGNAUX ERG PENDANT LE CHAUFFAGE RÉTINIEN ET DE RÉGULER LE CHAUFFAGE RÉTINIEN SUR LA BASE DE CES DERNIERS

(30) Priority: 10.09.2020 FI 20205875
(43) Date of publication of application: 19.07.2023
(73) Proprietor: Maculaser Oy, 02150 Espoo (FI)
(72) Inventor: KAIKKONEN, Ossi, 02150 Espoo (FI); TURUNEN, Teemu, 02150 Espoo (FI); KOSKELAINEN, Ari, 00076 Aalto (FI)
(74) Representative: Berggren Oy
(86) International application number: PCT/FI2021/050602
(87) International publication number: WO 2022/053745

(56) References cited:
- WO-A1-2019/197726
- WO-A1-2019/197726
- US-A1- 2018 289 265
- US-A1- 2020 069 463

## Description

### TECHNICAL FIELD OF THE INVENTION

The invention relates to electroretinography (ERG) and retinal heating in general. More specifically, the invention relates to obtaining ERG signals during retinal heating and controlling of the retinal heating based on the obtained ERG signals.

### BACKGROUND OF THE INVENTION

Electroretinography (ERG) is a method where electrical signals (electrical response) of the retina are recorded during exposure of the retina to stimuli, such as light flashes, which may be useful in various cases, such as in diagnosis of retinal diseases.

It has also been discovered that ERG signals obtained during retinal heating (e.g. photothermal retinal therapies) could be used to determine the temperature of the retina, due to the temperature dependence of the electrical signalling of the retina. An obtained ERG signal during retinal heating can therefore be indicative of a temperature (or at least a difference in temperature between two timepoints) that is occurring at the retina. The temperature determination related to heating could then be used e.g. for controlling the retinal heating so that the retinal temperature is kept at a desired level or a desired thermal dose is delivered during a retinal heating therapy procedure.

It is important to keep the retinal temperature at a level that is therapeutically effective while still avoiding temperatures that are high enough to cause damage to the retina. Prior art devices have not provided effective dosimetry to reach the optimal temperature and have not enabled the detection of overtreatment before damage occurs.

WO2019197726 discloses a device for heating therapy of the retinal pigment epithelium. Laser beams that are associated with separately adjustable optics are combined with beam splitters, where locations and sizes of laser spots can be adjusted independently once the beams reach the retina.

### SUMMARY OF THE INVENTION

The invention is defined in the appended claims.

An object of the invention is to alleviate at least some of the problems in the prior art. In accordance with one aspect of the present invention, an arrangement is provided for providing retinal ERG stimulus and heating, the arrangement comprising at least one processor and at least one light source for providing at least a stimulus beam to induce an ERG signal from a target area of the retina, the arrangement additionally comprising a heating system for elevating the temperature of at least the target area, wherein the processor is configured to receive a retinal ERG signal induced by the stimulus beam during retinal heating, and determine, based on said ERG signal, one or more indicators being indicative of a temperature of the retina, and control the heating system based on said indicator, said indicator comprising at least an amplitude of the ERG signal and/or the kinetics of the ERG signal, wherein the processor is additionally configured to compare the amplitude to a previously determined reference ERG response amplitude, and terminate or adjust the retinal heating and/or inform a user of the arrangement if the amplitude of the ERG signal during the retinal heating falls below a predetermined threshold relative to the previously determined reference ERG response amplitude, and/or in that the processor is configured to compare the kinetics of the ERG signal to a previously determined kinetics, and terminate or adjust the retinal heating and/or inform a user of the arrangement if the kinetics is slower than the previously determined kinetics by over a predetermined threshold amount.

Having regard to the utility of embodiments of the invention, retinal heating may be conducted more safely. Retinal heating may be controlled to elevate retinal temperature to a predetermined level, such as below a level of heating that could cause damage to the retina, but sufficiently high to induce therapeutic benefits.

One embodiment of the disclosure provides a device and method for carrying out a calibration protocol for determining temperature elevation of retinal tissue per unit heating power as an indicator. It has been noted by the inventors that the temperature elevation in the retina caused by the same laser power varies between patients, or even between different retinal areas. The disclosure provides a way of performing a power calibration and may therefore be used in such thermal dosimetry. Retinal heating may then be delivered in a way that is individually optimized for the treated area to reach the therapeutic temperature window. Based on the determined temperature elevation per unit heating power, the heating system can be controlled to provide a calibrated heating power to elevate the temperature of the retina, where the heating power is within a predetermined range to induce a predetermined temperature elevation of the retina in a specific case.

A power calibration protocol may comprise the processor being configured to:
a. initiate retinal stimulation by controlling the at least one light source to provide the stimulus beam
b. determine a first pre-heating reference ERG signal
c. initiate retinal heating by controlling the heating system to provide retinal heating to a target area with a first power and continue the retinal heating for a preset duration
d. determine a first heating ERG signal
e. optionally repeat steps c - d with a second or subsequent laser power to determine second or subsequent heating ERG signals
f. terminate the retinal heating
g. determine a first post-heating reference ERG signal
h. compare the first pre-heating ERG signals and/or first post-heating ERG signals to the heating ERG signals to determine the temperature elevation of the retinal tissue with the used heating power(s)
i. utilize the determined temperature elevation(s) to determine the temperature increase(s) of the target area per unit of heating power.

The processor may be configured to repeat at least steps c-d with a predetermined set of heating powers and based on the obtained plurality of temperature increases of the target area per unit of heating power, determine an aggregate temperature increase of the target area per unit of heating power as an indicator.

As the heating system is turned on, the temperature of the retina rises precipitously for the first seconds of heating, followed by a slow drift where the temperature changes slowly and the change in retinal temperature is stabilized (i.e. no longer changes as fast as during the first seconds of heating). In one embodiment, heating ERG signal(s) obtained between a predetermined time after changing or initiating retinal heating and a subsequent termination or change in retinal heating may be used as the heating ERG signals for temperature determination. Here, a temperature of the target area that better reflects or is more efficiently indicative of (closer to) the peak temperature elevation induced by the heat exposure may be obtained. The temperature elevation of the retina is expected to depend linearly on heating power, and the calibration procedure may extrapolate the required heating power by first determining the amount of heating power required per unit temperature elevation, and multiplying this number by the desired temperature elevation to determine the power required to reach the desired target temperature. To reach the target temperature of the retina, a heating power corresponding to a power required for increasing retinal temperature by an amount corresponding to a difference between a determined bodily temperature to the target temperature, the target temperature difference/elevation, may be applied.

ERG signals may, of course, be obtained/registered essentially continuously but it may be only those ERG signals obtained after the predetermined time delay which are determined as the heating ERG signals used in the determining of retinal temperature.

ERG signal(s) obtained essentially immediately after the heating is initiated may additionally or alternatively be used as the heating ERG signals for temperature determination to determine a rate of temperature increase in the target area of the retina caused by the heating at the beginning of the heating procedure. When the initial rate of temperature increase per unit heating power is known, the processor may determine an appropriate power and/or pulse duration to deliver a desired thermal dose. This can be done by presuming the temperature elevation to be directly proportional to laser power and pulse duration when the pulses are sufficiently short. If a different spot size is used in calibration and treatment, the treatment power may be adjusted in proportion to the diameter of the treatment spot.

In different embodiments of the disclosure where indicators are used to control the heating, the heating may be terminated or adjusted. Additionally or alternatively, the controlling of the heating may refer to a user of the arrangement being notified or informed. The informing may e.g. comprise providing auditory and/or visual information to the user that is indicative of a property of the heating, such as a temperature of the retina. Based on the informing, the user of the arrangement may be able to adjust or terminate the heating.

In one embodiment, the heating may be terminated or adjusted or a user may be informed if one or more indicators indicates that the temperature of the retina may be excessively high. As will be demonstrated, the indicator(s) may be related to changes in amplitude and/or kinetics of the recorded ERG response/signal obtained during heating either compared to an earlier previously determined reference ERG response recorded with an essentially corresponding heating power or an ERG response recorded prior to the heating. The one or more indicators may be used to determine a temperature of the retina or a temperature change of the retina and if this temperature differs from a target retinal temperature by over a threshold amount, this may a sign that the retinal temperature determined from the indicator is actually not accurate but the indicator, such as kinetics parameter, does not behave as it should in the target temperature range (e.g. acceleration of kinetics is not linear) and thus the retinal heating has advanced to temperatures outside of the therapeutic window and could be damaging.

The processor may additionally be configured to extrapolate a heating power that provides a predetermined temperature elevation in the target area. In cases where the core body temperature of the patient is determined as a part of a calibration protocol, the processor may be configured to determine a laser power that leads to a predetermined absolute temperature at the target area of the retina.

In one embodiment, the processor may extrapolate how the ERG signaling kinetics should change during the treatment with higher laser power, and may be configured to terminate the retinal heating or lower the treatment power if the change in kinetics deviates from the expectation more than a given threshold.

The processor may be configured to determine a signal-to-noise ratio of the obtained ERG signal and terminate the retinal heating by powering off the heating system or in a case where the retinal heating has not been initiated, the initiation of retinal heating may be disabled if the determined signal-to-noise ratio falls below a predetermined threshold value. The retinal heating may thus be prevented or aborted if the obtained ERG signal cannot effectively be used to obtain information relating to the target area. This may occur e.g. if an ERG electrode contact deteriorates or if the patient whose retina is being treated flexes their facial muscles. It may be safer to not perform retinal heat treatment in these cases.

A processor may in some embodiments be configured to receive or determine an impedance between ERG electrodes, such as the ocular electrode and reference electrode, as an indicator, and may terminate retinal heating or disable initiation of the retinal heating if the impedance is above a predetermined threshold value.

In one embodiment, a processor may be configured to determine an amplitude of the ERG signal as an indicator and terminate or adjust the retinal heating and/or inform a user if the amplitude during retinal heating falls below a predetermined threshold value with respect to a previously determined reference ERG signal amplitude. This may ensure that the retinal heating is terminated if the temperature of the retina becomes dangerously high, where the ERG signal amplitude begins to decrease excessively. Even in cases where a calibration protocol is carried out (e.g. in a manner disclosed later herein), the calibration protocol may have been erroneous, whereby monitoring of the ERG signal amplitude may be a safety mechanism for preventing excessively high retinal temperatures, which may be harmful for a patient.

The threshold relative amplitude between the amplitude of the ERG signal determined during the retinal heating and the amplitude of a previously determined reference ERG response amplitude may for instance be between 0.4 and 0.9, advantageously between 0.5 and 0.7.

In one more embodiment, the processor may be configured to determine the kinetics of the ERG signal as an indicator and terminate retinal heating if the said kinetics, such as an acceleration thereof, differs from a predetermined kinetics value by over a predetermined threshold amount. The ERG signal response kinetics is exponentially accelerating at a certain temperature range. In a configuration where impulse ERG responses are used for temperature determination, a temperature change depends on temperature in the following way: Δ*T*= *k ln(*Δ*x),* where Δ*T* is the change in temperature, Δ*x* is a relative change in a feature value describing the acceleration of the signal, and k is the temperature dependence of the feature.

In sufficiently high retinal temperatures, the temperature dependent acceleration no longer follows the aforementioned relationship, and when the temperature is sufficiently high, the response kinetics becomes slower with rising temperatures. If the signal kinetics slows excessively, e.g. slows by over a threshold amount compared to a reference value, this may be used to determine that the retinal temperature is excessively high. Monitoring kinetics of a determined ERG signal and subsequently terminating or adjusting retinal heating in the aforementioned case may therefore also provide a safety mechanism that may prevent harmful heating of the retina.

In one embodiment of the disclosure, the deceleration of ERG signaling kinetics due to excessively high temperatures may be detected by essentially continuously recording the ERG signal during retinal heating and comparing the most recent ERG response signal to the fastest ERG response recorded previously during the retinal heating involving substantially the same heating power. A threshold amount of slowing in kinetics may be determined to correspond to a change of temperature between 0.5°C to 4°C, advantageously between 1°C to 2°C.

The relationship between kinetics of the ERG signal and change in retinal temperature may vary slightly based on the chosen method of retinal stimulation. With preknown data, the temperature dependence of ERG signaling kinetics acceleration was found to be 3.6%/°C when bright photopic flashes are used in retinal stimulation over a bright constant background, which was found to be an advantageous method for eliciting and ERG signal during laser heating application. However, with other stimulation paradigms, this value might vary e.g. in the range of 3% - 6% /°C.

The inventors have discovered that a drop in ERG signal amplitude as well as deceleration of ERG kinetics occur below retinal temperatures where damage occurs and above retinal temperatures where therapeutic benefits are triggered. When using a 1 minute laser exposure, increased production of molecular chaperones HSP70 and HSP90, which are believed to relay the therapeutic effect of retinal laser therapy, were detected in treatments where the retinal temperature stabilized above 44°C, while the damage threshold was determined to be approximately 48°C.

It may be determined, e.g. via a calibration protocol, that a known kinetics acceleration, e.g. given by equation ΔT=k In(Δx), of the ERG signal may be expected. If it is observed during heating of the retina that the kinetics of the ERG signal accelerate an amount less than this predetermined expected acceleration value by more than a threshold amount, it may be an indicator that the temperature has risen to a point where the aforementioned ΔT= k In(Δx) relationship does not hold.

In one embodiment, an indicator may be a kinetics parameter of the ERG signal, where the change in ERG response kinetics induced by e.g. laser exposure with a known target temperature of the retina is compared to the expected kinetics acceleration from the aforementioned equation ΔT=kln(Δx). If the change in the acceleration of ERG response kinetics is significantly less than this expectation, it is an indication that the temperature has risen excessively high, i.e. over the target temperature or beyond a therapeutic window. The threshold for the aforementioned difference may correspond to a temperature decrease of preferably between 2°C and 8°C, advantageously between 4°C and 6°C.

The inventors have also discovered that an optimal treatment temperature for retinal heating is in a temperature range where an obtained ERG signal kinetics is at its fastest, i.e. when the temperature is either increased or decreased. When retinal temperature rises above the optimal treatment temperature, ERG signaling starts to decelerate rapidly. This rapid deceleration of ERG signaling kinetics was found to precede retinal damage, making it suitable for detecting inadvertent overtreatment.

The inventors have yet additionally discovered that the amplitude of the ERG signal does not exhibit dramatic changes at the optimal treatment temperature, but the amplitude starts to drop rapidly when the retinal temperature is increased beyond the optimal treatment temperature. This rapid drop in ERG amplitude was found to precede retinal damage, making it suitable for detecting inadvertent overtreatment.

The exemplary embodiments presented in this text are not to be interpreted to pose limitations to the applicability of the appended claims. The verb "to comprise" is used in this text as an open limitation that does not exclude the existence of unrecited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the disclosure will be described in greater detail with reference to exemplary embodiments in accordance with the accompanying drawings, in which:
Figure 1 shows a portion of an exemplary arrangement according to an embodiment of the disclosure in connection with a device for conducting retinal heating while obtaining an ERG signal,
Figure 2 illustrates one schematic view of an exemplary arrangement according to one embodiment of the disclosure
Figure 3 shows a plot with determined relative amplitude between a determined ERG signal compared to a reference ERG signal as a function of retinal temperature, and
Figure 4 shows a plot with determined temperature difference between a target temperature and a temperature determined from an ERG signal as a function of retinal temperature.

### DETAILED DESCRIPTION

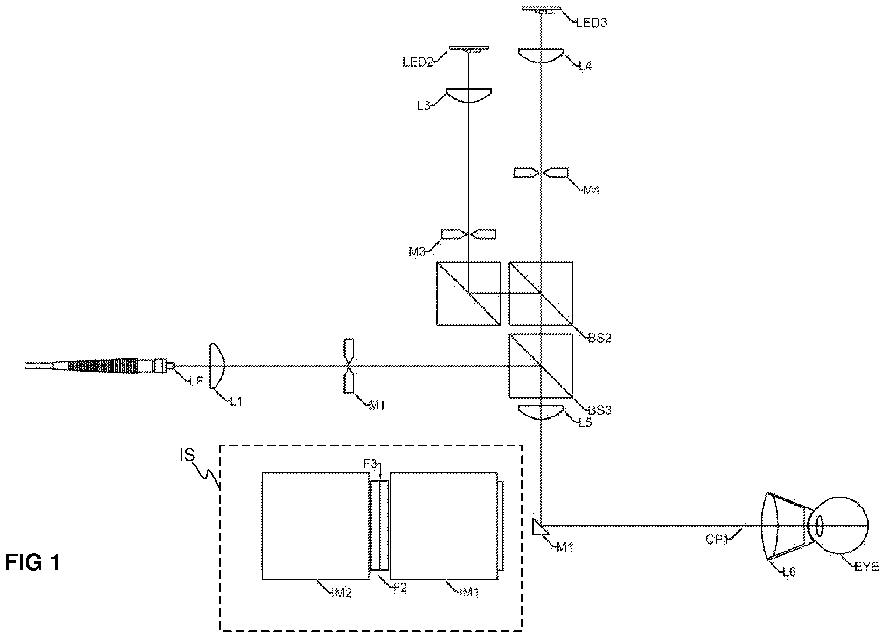

Figure 1 shows a device for obtaining retinal ERG signals during retinal heating, where the device comprises constituents of which at least a portion are contained in an arrangement according to embodiments of the disclosure.

The device comprises at least one light source, where the at least one light source is configured to provide at least a stimulus beam for illuminating a target area of the retina to stimulate the target area of the retina to elicit a focal ERG signal . Also other light sources or light beams, such as a central background beam for illuminating at least the target area of the retina may be optionally provided in a device. The light sources (in the case of a plurality of such) are preferably controllable separately. The device additionally comprises a heating system for elevating the temperature of the retina.

In the embodiment of Fig. 1, the stimulus beam is provided by a stimulus light source LED3. The stimulus beam is used to illuminate at least a target area of the retina, where the target area is an area that is an area from which ERG signals are to be obtained and optionally also an area which is heated/to be heated during retinal heating. The target area may refer to the area that is heated, while the area illuminated by the stimulus beam in this case may not correspond to the entire target area. The stimulus beam is used to elicit/induce an ERG signal from the target area. The stimulus beam light source LED3 may be a light-emitting diode (LED) light source configured to provide a stimulus beam having wavelength of 500 - 600 nm, e.g. about 555 nm. Red and green cone cells have similar sensitivity at a wavelength of 555 nm, therefore a stimulus beam exhibiting a wavelength close to this may stimulate both cell types similarly. The stimulus beam may comprise white light, which may stimulate all retinal cone cells equally.

The stimulus light source LED3 may be configured to provide a stimulus light beam that is modulated. The stimulus beam therefore may not be provided as a continuous beam of light, but may comprise sequences, such as impulse-like flashes of light. The modulation may be implemented e.g. at a frequency between 4 and 40 Hz, advantageously between 10 and 25 Hz.

The device of Fig. 1 is shown in connection with a fundus imaging system IS (which may in some embodiments also be realized as part of a device). The fundus imaging system IS may for instance be eyepieces, a fundus camera or a scanning laser ophthalmoscope. The fundus imaging system may comprise a first imaging module IM1 and second imaging module IM2 and one or more filters, such as a second filter F2 (for instance optical filter) and third filter F3, e.g. an infrared-cut-filter, which blocks laser light from being directed to imaging module IM2.

In the device of Fig. 1, a central background light beam is provided by a central background light source LED 2. The central background light beam is configured to illuminate at least the target area of the retina and maintain a light adaptation level of the target area. The central background light beam may be concentric to the target area and may be limited to essentially illuminating the target area only. The central background light source LED 2 may be an LED light source. The central background light beam may comprise white light and/or may have a brightness of >100 lux at the fundus.

The brightness of the central background beam may be configured to be reduced as a heating laser or other heating means is turned on, to maintain a steady illuminance at the target area.

In use case scenarios where e.g. infrared imaging is used for fundus imaging, the central background light beam may not be needed.

A device also comprises means for obtaining an ERG signal, i.e. means for obtaining a response signal of the target area to the stimulus provided by the stimulus beam. The ERG signal may be an electrical response that may be recorded/collected or obtained by one or more ERG electrodes. The electrodes may comprise one or more ocular electrodes and one or more reference electrodes. The ERG signal may be obtained as a voltage change between at least two electrodes over time.

The device may comprise or be usable in connection with a fundus lens L6. The fundus lens L6 may direct the provided light beams to the fundus of the eye. The fundus lens L6 may for instance be an inverting fundus lens with a field of view >120 degrees.

In one embodiment, a fundus lens may be integrated into the device, whereby a lens is not required to be placed on the cornea.

In one embodiment, a device comprises a fundus lens and ocular electrodes may be integrated into the fundus lens.

A heating system comprises at least a heat source, such as heating laser LF that is configured to elevate the temperature of the target area on the fundus. A heating light source LF may be configured to provide a heating beam that is directed to at least the target area.

The heating beam may comprise wavelength in the near-IR area. Wavelengths of light comprised in the heating beam may be 700-1000 nm, while the heating beam may be provided by a heating light source LF that is a fiber coupled diode laser.

In one embodiment, a heating beam has a homogenous irradiance profile and spot diameter of 1-6 mm on the fundus.

Also other heating systems or means for heating may be utilized in connection with devices that are associated with retinal heating. For instance, the heating system may be implemented through ultrasound.

Considering the functioning of the device shown in Fig. 1 in connection with an arrangement, one use case scenario is described next. The three beams discussed above may be brought onto the same channel and projected onto the conjugate plane CP1. Three light channels may thus be involved, corresponding to the stimulus beam, central background light beam, and heating beam. The heating beam may be directed onto a first mask M1 by a first lens L1. The central background light beam may be directed onto a third mask M3 by a third lens L3. A stimulus beam may be directed onto a fourth mask M4 by a fourth lens L4.

The fundus lens L6 may project the light profile at CP1 onto the fundus. The first imaging module IM1 is configured such that CP1 is either projected onto a camera sensor, or that the eyes of the treating physician are able to focus onto CP1 through biomicroscope eyepieces. The beams passed through a fifth lens L5 are directed towards the eye by a first mirror M1. The first mirror M1 may be placed directly in front of the first optics module IM1 (such as biomicroscope), so that the left eye has a view to the fundus on the left side of the first mirror M1, and the right eye has a view to the fundus on the right side of the first mirror M1. The fifth lens L5 may project images from masks M1, M3 and M4 onto the conjugate plane CP1, meaning that the light profile emitted through the masks are imaged onto CP1. Beam splitters BS2 and BS3 may combine beams arising from the heating laser fiber output LF, and light sources LED2, and LED3.

In Fig. 1 the masks M1, M3, M4 are holes and a light profile transmitted through the hole is projected onwards to the conjugate plane CP1. The masks may also be shaped mirrors or digital micromirror devices, in which case the light is made to reflect from the mask instead of passing through it.

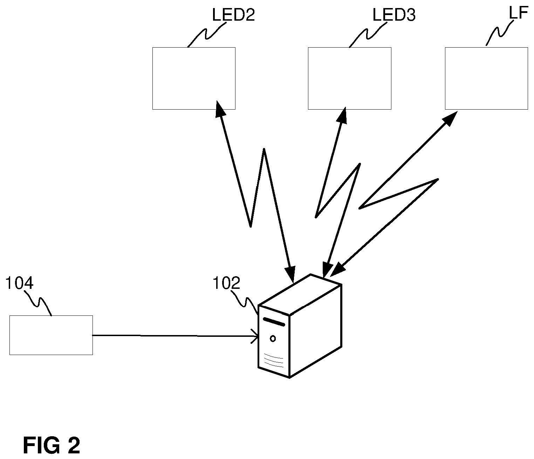

Figure 2 illustrates schematically one exemplary arrangement according to an embodiment of the disclosure The arrangement comprises at least one processor 102 and at least one light source for providing at least a stimulus beam, the arrangement additionally comprising a heating system for elevating the temperature of the target area of the retina. An arrangement may comprise or be used with also other components, such as those depicted in Fig. 1.

Preferably, an arrangement comprises at least one processor 102, a stimulus light source LED3, and a heating light source LF (or some other heating system). The arrangement may also comprise a central background light source LED2. The processor 102 is configured to control some or all of the light sources (and/or other heating system). Controlling may be powering on and off of the light sources or controlling of any other aspect of the light sources, such as power or illuminance delivered by a provided light beam. The processor 102 is configured to receive obtained ERG signals, which may be obtained by electrodes 104. The electrodes 104 may comprise a plurality of electrodes, such as at least one ocular electrode and at least one reference electrode. The processor 102 may be configured to analyze the obtained ERG signals and determine one or more related parameters. Electrodes 104 may in some embodiments be comprised in the arrangement.

The ERG signals obtained from the electrodes 104 may be directed through an ERG amplifier-digitizer system for processing, such as amplification, filtering and digitizing, before being received at the processor 102. Such an ERG amplifier-digitizer system may in some embodiments be considered as part of the arrangement, in some embodiments part of the processor 102.

The processor 102 may be configured to initiate retinal heating by powering on a heating light source LF. The processor may be configured to initiate stimulation of a target area of the retina using the stimulus beam by powering on the stimulus light source LED3. The processor 102 may then be configured to receive retinal ERG signals induced by the stimulus beam during retinal heating and determine, based on the obtained ERG signal, one or more indicators being indicative of a temperature of the retina, and control the heating system based on said indicator.

The controlling of the heating system may comprise controlling the heating system such as heating light source LF to provide a determined heating power (could also mean essentially no power) to provide a retinal temperature that is within a predetermined range, such that the retinal temperature may remain at a safe and/or therapeutic range and/or be prevented from rising above a certain threshold to retain safety of the retinal heating.

In one embodiment, the arrangement may be configured to determine that retinal heating is being conducted in a temperature range where the ERG signal kinetics accelerate exponentially with rising temperatures. A heating system may be controlled to e.g. terminate or adjust heating if it is determined that the signal kinetics determined from the ERG signal accelerates an amount that is significantly different from what is expected based on the target temperature elevation.

In the temperature range where the ERG signal kinetics depend exponentially on temperature, the amplitude is not highly sensitive to temperature changes. At higher temperatures, the amplitude starts to reduce with higher temperatures, which may be used as an indicator for terminating the treatment or adjusting the heating power.

A calibration protocol may be conducted with one or more heating powers resulting in temperature elevations within this range (the range where the ERG signal kinetics accelerate exponentially with rising temperatures).

The processor 102 may be configured to determine a signal-to-noise ratio of the obtained ERG signal and terminate the retinal heating by powering off the heating light source LF or in a case where the retinal heating has not been initiated, the initiation of retinal heating may be disabled if the determined signal-to-noise ratio falls below a predetermined threshold value. The determining of the signal-to-noise ratio may be conducted essentially continuously. The predetermined threshold value may be a value for the signal-to-noise ratio where obtaining of a reliable ERG signal is not feasible.

A processor 102 may in some embodiments be configured to determine an impedance between ERG electrodes, such as the ocular electrode and reference electrode, based on the received ERG signal and may terminate retinal heating or disable initiation of the retinal heating if the impedance is above a predetermined threshold value.

In one embodiment, a processor 102 may be configured to determine an amplitude of the ERG signal and terminate the retinal heating if the amplitude during retinal heating falls below a predetermined threshold value with respect to a previously determined reference ERG signal amplitude.

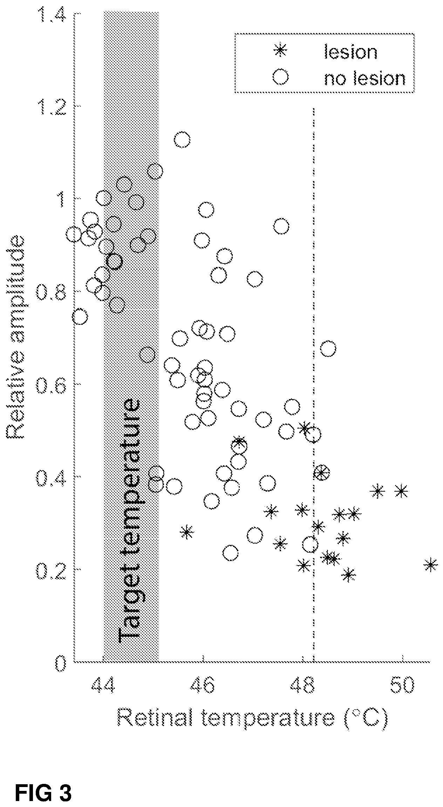

Fig. 3 shows a determined relative amplitude between a determined ERG signal compared to a reference ERG signal on the vertical axis and experimental target retinal temperature on the horizontal axis. A target temperature range is shown as 44-45 °C, which may be considered as a therapeutic window for treatment in a normal use case. The relative amplitude refers to a value of a determined ERG signal during heating divided by an ERG signal that has been determined in a reference case, preferably with the same heating power or directly before heating such as laser application. The points in the figure represent unique treatments performed in a preclinical trial, where a calibration procedure was performed to determine the appropriate laser power for the treatment to reach the experimental target temperature. Treatments that resulted in retinal coagulation (lesion) are marked with an asterisk and treatments without coagulation are marked with a circle. The treatments marked with an asterisk may thus induce damage to the retina and in these cases the provided heating power may be too high.

It may be seen from Fig. 3 that as an example, if a threshold of 0.6 for the relative amplitude is used to terminate the treatment, the treatment is rarely terminated when the treatment temperature is below 45°C and reliably terminated in cases where the laser exposure would cause cellular damage.

An arrangement may be configured to perform a power calibration protocol for determining a heating power that corresponds to a desired retinal temperature or change in retinal temperature. Temperature elevation per unit heating power for the target area, may be determined as an indicator that may be used to control the heating system to provide a calibrated heating power to elevate the temperature of the retina. The power calibration protocol may for instance be carried out before performing of a retinal heating treatment, where the processor 102 may be configured to
a. initiate retinal stimulation by controlling the at least one light source to provide the stimulus beam
b. determine a first pre-heating reference ERG signal
c. initiate retinal heating by controlling the heating system to provide retinal heating to a target area with a first power and continue the retinal heating for a preset duration
d. determine a first heating ERG signal after the change in retinal temperature has stabilized (e.g. after a predetermined time duration such as 10-20 seconds, after which it is known or assumed that the temperature change has slowed significantly)
e. optionally repeat steps c - d with a second or subsequent laser power to determine second or subsequent heating ERG signals
f. terminate the retinal heating
g. optionally determine a first post-heating reference ERG signal after the retinal temperature has returned to body temperature (e.g. after a predetermined time duration after which it is known or assumed that the temperature has decreased to the initial body temperature)
h. compare the first pre-heating ERG signals and/or first post-heating ERG signals to the heating ERG signals to determine the temperature elevation of the retinal tissue with the used heating power(s)
i. utilize the determined temperature elevation(s) to determine the temperature increase(s) of the target area per unit of heating power. Some alternative ways of determining the temperature elevation of the retinal tissue with the used heating power based on comparison of the pre-heating ERG signals and/or post-heating ERG signals to the heating ERG signals will be described later herein.

The processor 102 may be configured to repeat at least the above mentioned steps c-d with a predetermined set of heating powers and based on the obtained plurality of temperature increases of the target area per unit of heating power, determine an aggregate temperature increase of the target area per unit of heating power. For instance, a zero intersecting linear fit between heating power and temperature elevation may be utilized, the temperature increase per unit power being the slope of the linear fit.

In some embodiments, heating ERG signals may be selected to exclude the ERG responses within a predetermined time duration after initiating or changing retinal heating and only include responses obtained after said time duration and before a subsequent change or termination of retinal heating. Here, it may be known or determined that a change in retinal temperature has stabilized or changes slowly (increases slowly towards a peak retinal temperature) after the predetermined time duration. The change in retinal temperature may occur slowly after the predetermined time period, e.g. after the first seconds of heating as compared to the change in retinal temperature occurring during the first seconds of heating, where during these first seconds of heating, the change in retinal temperature is faster. Utilizing ERG responses obtained after said predetermined time duration for temperature determination may result in a determined retinal temperature that is closer to an actual peak retinal temperature that is caused by the heating power used.

Through a calibration protocol and with a determined or estimated body temperature and a determined target retinal temperature, a target temperature elevation of the retinal tissue may be determined and a corresponding heating power may be delivered. However, even in cases where a calibration protocol is carried out, errors in the determined temperature increase of the target area per unit of heating power and/or in body temperature may lead to an actual elevation of retinal tissue that is excessive, and one or more additional indicators, such as kinetics of the ERG signal may be used to control the heating to avoid overtreatment.

When a calibration protocol is used to determine a heating power that should be used to reach a target temperature (or temperature elevation) of the retina, a retinal temperature (elevation) determined from a continuously recorded ERG signal may indicate overtreatment if it is significantly below the target temperature (elevation) during heating exposure.

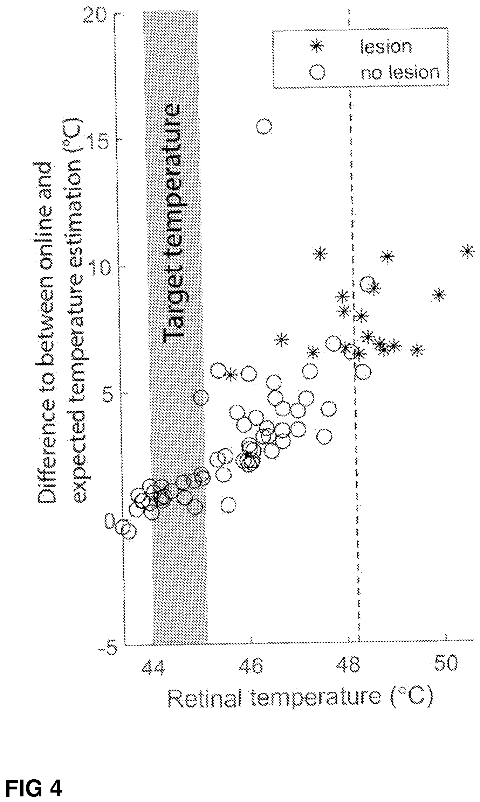

Figure 4 shows determined temperature difference between a target temperature determined through a calibration protocol and a temperature determined based on a determined ERG signal during heating on the vertical axis and an experimental target retinal temperature on the horizontal axis. It is seen that if as an example, a threshold of 5°C difference between the target temperature and temperature determined during the heating is used, the treatment is rarely terminated when the temperature is below 45 and reliably triggered in treatments that would cause cellular damage.

In one more embodiment, the processor 102 may be configured to determine the kinetics of the ERG signal and e.g. terminate or adjust retinal heating if the acceleration of said kinetics differs from a predetermined acceleration value by over a predetermined threshold amount. In one embodiment, the predetermined acceleration value is determined based on a calibration protocol.

The kinetics may comprise e.g. speed or rate or response time of the obtained/measured ERG signal (electrical response) of the retinal tissue to the stimulation by the stimulation beam. Features may be extracted from ERG signals to reflect changes in response kinetics and therefore changes in retinal temperature. In embodiments where the impulse ERG response is used for temperature determination, these features may be e.g. relative changes in time-to-peak of the b-wave relative to the moment of the impulse stimulus (ratio of time-to-peak values between two responses) or the overall time axis compression applied on an ERG response to maximize the similarity with another response. A logarithm of these features may be used as an indicator that depends linearly on temperature differential between the compared responses.

As the retinal temperature increases mildly, the kinetics of the ERG signal accelerate in a way that implicit times in the ERG signal shift towards the time of the stimulus an amount that is proportional to the logarithm of the temperature change of the retina. When the retinal temperature increases above 44°C, this relationship breaks and the ratio between kinetics acceleration and temperature change first reduces, and when the temperature increases further, the kinetics start to decelerate with rising temperatures. This effect can be used as a safety mechanism to terminate the treatment or lower e.g. the laser power. The kinetics may be used as a safety mechanism with or without a calibration protocol.

The deceleration of ERG kinetics in high temperatures may also be used as an indicator of overtreatment by continuously recording ERG during e.g. laser exposure and terminating the treatment if the kinetics of the ERG signal start to decelerate during the laser exposure. This can be implemented by comparing the kinetics of the continuously updated or recorded ERG response during heating to a reference ERG response with the fastest kinetics that has been previously obtained during the same heating power exposure.

It may be determined that the kinetics of an ERG response accelerates 3-6%, e.g. about 3.6%, when then temperature of the retina is increased by 1°C. In one exemplary scenario, a heating treatment may be performed where at time t=0, the kinetics has accelerated by 0% at time t=5s, the kinetics has accelerated by 10%, at time t=10s, the kinetics has accelerated by 20%, at time t=15s, the kinetics has accelerated by 21%, and at time t=20s, the kinetics has accelerated by 15%. With the fastest kinetics, the kinetics accelerated by 21% and after this the kinetics slowed by 6%, which may translate to a temperature change of the retinal tissue of slightly under 2°C. An increase in temperature may be estimated to be exceedingly high, because the acceleration of the kinetics slowed into deceleration during the treatment. In a normal or intended treatment session where the temperature of the retina is maintained at or near a target temperature or therapeutic window, the kinetics should accelerate and approach a stable value until treatment is terminated.

If the ERG response has decelerated an amount corresponding to, e.g., more than 1°C of temperature compared to the fastest ERG response recorded during the same heating procedure, it may serve as an indicator for increased risk of overtreatment.

In one example, a calibration protocol may be carried out to determine a heating unit power that is required for temperature elevation of one unit, such as 1°C. E.g. if the target temperature elevation is determined as 8°C, then it is expected that 8 units of heating power are required. If during the treatment it is observed that the ERG signal kinetics is for example 10% accelerated as compared to a situation without retinal heating, which would be expected to correspond to about 3°C temperature change, then it may be determined that the actual temperature change of the retina is much higher, as the temperature has already moved to an area where the ERG signal kinetics has decelerated.

In some embodiments, a temperature of the retina may be determined by determining a core body temperature of the patient using known methods, determining a temperature increase that is or may be achieved at the target area of the retina through e.g. a calibration procedure as above (and determining a temperature increase corresponding to a certain heating power), and then adding the determined temperature increase to the core body temperature to obtain a total temperature of the retinal tissue at the target area.

In one embodiment, the power calibration protocol may be performed in a temperature range where there is an exponential relationship between temperature and acceleration of signaling kinetics and a predetermined set of heating powers used may be e.g. 20%, 35%, and 50% of an assumed or initially estimated heating power that may be used to induce a desired temperature elevation.

In one embodiment, a confidence interval or error parameter may also be determined. The arrangement may be configured to repeat the calibration protocol or some steps of it until e.g. the confidence interval is sufficiently narrow, such as below some predetermined value.

The kinetics of the ERG signal accelerates with higher temperatures near the normal body temperature. The temperature dependent acceleration of ERG responses can be used for determining the temperature elevation of the retina caused by the application of retinal laser therapy/heating of the target area. Before retinal laser therapy is initiated, the retina is near core body temperature. ERG responses recorded without retinal heating thus serve as a reference for ERG signaling when the retina is at normal body temperature.

When heating is applied to the retina, the temperature of the retinal tissue increases and ERG responses recorded during heating represent ERG signaling at an elevated temperature compared to normal body temperature, and changes in signaling kinetics can be used to determine the amount of temperature elevation caused by e.g. laser heating.

There are various methods for determining retinal temperature from the ERG response, and the accuracy of temperature determination depends both on the stimulation protocol used to elicit the ERG signal and the algorithm used to determine temperature from the signals. Different stimulation protocols also produce different ERG responses, and the temperature determination method may be tailored for the type of retinal stimulation used.

One set of retinal temperature determination methods relates to analyzing the impulse ERG-response. The impulse ERG response can be obtained, for example, by stimulating the target area repeatedly with flashes of light and averaging the responses between two flash stimuli. Another way for obtaining the impulse response is to stimulate the target area of the retina with light modulated by white noise, determining the transfer function between the light stimulus and the ERG response, and using the transfer function to generate the impulse response.

One exemplary stimulation protocol for determining the temperature difference between two ERG impulse responses is by analyzing changes in kinetics, such as time delays of the ERG impulse response. The ERG impulse response typically has one or more peaks, and the time delay between the flash stimulus and the signal peak is an example of a signal feature that can be used in temperature determination. The amount of time shift in the peak(s) between ERG impulse responses recorded with and without (laser) heating is/are proportional to the extent of temperature elevation in the retina, and can be used to determine changes in retinal temperature.

Another exemplary method for determining the temperature difference from two ERG impulse responses works by compressing/expanding the time axis of ERG signal of one or both of them, with the compression origin set to the time of the impulse stimulus, and determining the amount of time-axis compression/expansion that maximizes the correlation between the responses. The amount of time axis compression/expansion producing the maximum correlation between the responses can be used to determine the amount of temperature elevation caused by the retinal heating and can be used to determine changes in retinal temperature of the target area.

Another proposed method for ERG-based retinal temperature determination works by stimulating the target area of the retina with a square wave and analyzing the resulting ERG signal in the frequency domain. The acceleration of signaling kinetics is reported to translate to a time shift in the frequency band of the ERG signal corresponding to the frequency of the square wave stimulus, and may reportedly be used to determine changes in retinal temperature of the target area.

The features recited in dependent claims are mutually freely combinable unless otherwise explicitly stated.

## Claims

1. An arrangement for providing retinal electroretinography, ERG, stimulus and heating, the arrangement comprising at least one processor (102) and at least one light source (LED3) for providing at least a stimulus beam to induce an ERG signal from a target area of the retina, the arrangement additionally comprising a heating system for elevating the temperature of at least the target area, wherein the processor is configured to receive a retinal ERG signal induced by the stimulus beam during retinal heating, determine, based on said ERG signal, one or more indicators being indicative of a temperature of the retina, and control the heating system based on said indicator said indicator comprising at least an amplitude of the ERG signal and/or the kinetics of the ERG signal,
**characterized in that**:
the processor is additionally configured to compare the amplitude to a previously determined reference ERG response amplitude, and terminate or adjust the retinal heating if the amplitude of the ERG signal during the retinal heating falls below a predetermined threshold relative to the previously determined reference ERG response amplitude and/or inform a user of the arrangement that the amplitude of the ERG signal during the retinal heating falls below a predetermined threshold relative to the previously determined reference ERG response amplitude,
and/or **in that** the processor is configured to compare the kinetics of the ERG signal to a previously determined kinetics, and terminate or adjust the retinal heating if the kinetics is slower than the previously determined kinetics by over a predetermined threshold amount and/or inform a user of the arrangement that the kinetics is slower than the previously determined kinetics by over a predetermined threshold amount.

2. The arrangement of claim 1, wherein the arrangement is configured to run a calibration protocol and determine, as an indicator, the temperature elevation per unit heating power for the target area of the fundus, and use said indicator to control the heating system to provide a calibrated heating power to elevate the temperature of the retina.

3. The arrangement of claim 2, wherein the processor is configured to:
a. initiate retinal stimulation by controlling the at least one light source to provide the stimulus beam
b. determine a first pre-heating reference ERG signal
c. initiate retinal heating by controlling the heating system to provide retinal heating to a target area with a first power and continue the retinal heating for a preset duration
d. determine a first heating ERG signal
e. optionally repeat steps c - d with a second or subsequent power to determine second or subsequent heating ERG signals
f. terminate the retinal heating
g. optionally determine a first post-heating reference ERG signal
h. compare the first pre-heating ERG signal and/or first post-heating ERG signal to the first heating ERG signal to determine the temperature elevation of the retinal tissue with the first power, and if steps c - d are repeated with a second power, compare the first pre-heating ERG signal and/or first post-heating ERG signal to the second heating ERG signal to determine the temperature elevation of the retinal tissue with the second power, and if steps c - d are repeated with a subsequent power, compare the first pre-heating ERG signal and/or first post-heating ERG signal to the subsequent heating ERG signal to determine the temperature elevation of the retinal tissue with the subsequent heating power
i. utilize the determined temperature elevation of the retinal tissue with the first power to determine the temperature increase of the target area per unit of heating power, and if temperature elevation of the retinal tissue is determined with a second power, utilize the determined temperature elevation of the retinal tissue with the second power to determine the temperature increase of the target area per unit of heating power, and if steps c - d are repeated with a subsequent power, utilize the determined temperature elevation of the retinal tissue with the subsequent power to determine the temperature increase of the target area per unit of heating power.

4. The arrangement of claim 3, wherein the processor is configured to repeat at least steps c-d with a predetermined set of heating powers and based on the obtained plurality of temperature increases of the target area per unit of heating power, determine, as an indicator, an aggregate temperature increase of the target area per unit of heating power.

5. The arrangement of claim 3 or 4, wherein the processor is configured to use ERG signals obtained between a predetermined time after changing or initiating retinal heating and a subsequent termination or change in retinal heating as the heating ERG signals for temperature determination.

6. The arrangement of claim 3 or 4, wherein the processor is configured to use ERG signals obtained essentially immediately after the heating is initiated as the heating ERG signals for temperature determination to determine a rate of temperature increase in the target area of the retina caused by the heating at the beginning of the heating procedure.

7. The arrangement of any previous claim, wherein the processor is configured to run a calibration protocol to determine a heating power that is expected to elevate the temperature of at least the target area to a target temperature or temperature elevation, and the processor is configured to continuously determine a kinetics parameter of the ERG signal during the retinal heating by the arrangement providing the heating power based on said calibration protocol, and the processor is further configured to determine a change in kinetics parameter of the ERG signal during the retinal heating and compare the change in kinetics parameter to an expected change in kinetics parameter based on a determined target temperature or temperature elevation, wherein the arrangement is configured to terminate or adjust the heating if the determined change in kinetics parameter differs from the expected change in kinetics parameter by over a threshold amount and/or wherein the arrangement is configured to inform a user of the arrangement if the determined change in kinetics parameter differs from the expected change in kinetics parameter by over a threshold amount.

8. The arrangement of claim 7, wherein the threshold amount of the difference between expected and observed change in kinetics parameter corresponds to between 2°C and 8°C, advantageously between 4°C and 6°C of temperature change.

9. The arrangement of any previous claim, wherein the processor is configured to extrapolate a heating power that provides a predetermined temperature elevation at the target area or a predetermined absolute temperature at the target area in cases where a body temperature is determined.

10. The arrangement of claim 5, wherein the processor is configured to extrapolate how the ERG signaling kinetics should change during the treatment with higher power and is configured to terminate the retinal heating or lower the treatment power if the change in kinetics deviates from the expectation over a predetermined amount.

11. The arrangement of any previous claim, wherein the processor is configured to determine the signal-to-noise ratio of the ERG signal and terminate the retinal heating if the signal-to-noise ratio is below a predetermined threshold value.

12. The arrangement of any previous claim, wherein the threshold relative amplitude between the amplitude of the ERG signal determined during the retinal heating and the amplitude of a previously determined reference ERG response amplitude is between 0.4 and 0.9, advantageously between 0.5 and 0.7.

13. The arrangement of any previous claim, wherein the processor is configured to determine the threshold amount of change in kinetics as corresponding to a change of temperature between 0.5°C and 4°C, advantageously between 1°C and 2°C, wherein the processor is configured to determine a relationship between kinetics and temperature based on a previously determined change in kinetics per one degree of temperature change in a predetermined therapeutic window of retinal temperature.

14. The arrangement of any previous claim, wherein the processor is configured to determine, as an indicator, the impedance between ERG electrodes based on said obtained ERG signal and terminate or disable the initiation of retinal heating if the impedance is above a predetermined threshold value.

## Patentansprüche

1. Anordnung zur Bereitstellung von Stimuli und Erwärmung für die Elektroretinographie, ERG, der Netzhaut, die Anordnung umfassend einen Prozessor (102) und mindestens eine Lichtquelle (LED3), um mindestens einen Stimulusstrahl bereitzustellen, der ein ERG-Signal aus einem Zielbereich der Netzhaut induziert, die Anordnung zusätzlich umfassend ein Erwärmungssystem zum Erhöhen der Temperatur zumindest des Zielbereichs, wobei der Prozessor so konfiguriert ist, dass er ein durch den Stimulusstrahl während der Erwärmung der Netzhaut induziertes Netzhaut-ERG-Signal empfängt, basierend auf dem ERG-Signal einen oder mehrere Indikatoren bestimmt, die eine Temperatur der Netzhaut anzeigen, und das Erwärmungssystem basierend auf dem Indikator steuert, der Indikator umfassend mindestens eine Amplitude des ERG-Signals und/oder die Kinetik des ERG-Signals.
**dadurch gekennzeichnet, dass:**
der Prozessor zusätzlich so konfiguriert ist, dass er die Amplitude mit einer vorstehend bestimmten Referenz-ERG-Reaktionsamplitude vergleicht und die Erwärmung der Netzhaut beendet oder anpasst, wenn die Amplitude des ERG-Signals während der Erwärmung der Netzhaut unter einen vorgegebenen Schwellenwert relativ zur vorstehend bestimmten Referenz-ERG-Reaktionsamplitude fällt, und/oder einen Benutzer der Anordnung darüber informiert, dass die Amplitude des ERG-Signals während der Erwärmung der Netzhaut unter einen vorgegebenen Schwellenwert relativ zur vorstehend bestimmten Referenz-ERG-Reaktionsamplitude fällt.
und/oder dass der Prozessor so konfiguriert ist, dass er die Kinetik des ERG-Signals mit einer vorstehend bestimmten Kinetik vergleicht und die Erwärmung der Netzhaut beendet oder anpasst, wenn die Kinetik um mehr als einen vorgegebenen Schwellenwert langsamer ist als die vorstehend bestimmte Kinetik, und/oder einen Benutzer der Anordnung darüber informiert, dass die Kinetik um mehr als einen vorgegebenen Schwellenwert langsamer ist als die vorstehend bestimmte Kinetik.

2. Anordnung nach Anspruch 1, wobei die Anordnung so konfiguriert ist, dass sie ein Kalibrierungsprotokoll ausführt und als Indikator die Temperaturerhöhung pro Einheit der Erwärmungsleistung für den Zielbereich des Fundus bestimmt und diesen Indikator verwendet, um das Erwärmungssystem so zu steuern, dass eine kalibrierte Erwärmungsleistung bereitgestellt wird, um die Temperatur der Netzhaut zu erhöhen.

3. Anordnung nach Anspruch 2, wobei der Prozessor so konfiguriert ist, dass er:
a. die Netzhautstimulation durch Steuerung der mindestens einer Lichtquelle initiiert
um den Stimulationsstrahl bereitzustellen
b. ein erstes Vorerwärmungs-Referenz-ERG-Signal bestimmt
c. die Erwärmung der Netzhaut initiiert, indem er das Erwärmungssystem so steuert, dass eine Erwärmung der Netzhaut in einem Zielbereich mit einer ersten Leistung bereitgestellt wird, und die Erwärmung der Netzhaut für eine voreingestellte Dauer fortsetzt
d. ein erstes Erwärmungs-ERG-Signal bestimmt
e. optional die Schritte c-d mit einer zweiten oder nachfolgenden Leistung wiederholt, um zweite oder nachfolgende Erwärmungs-ERG-Signale zu bestimmen
f. die Erwärmung der Netzhaut beendet
g. optional ein erstes Nacherwärmungsreferenz-ERG-Signal bestimmt
h. das erste Vorerwärmungs-ERG-Signal und/oder das erste Nacherwärmungs-ERG-Signal mit dem ersten Erwärmungs-ERG-Signal vergleicht, um den Temperaturanstieg des Netzhautgewebes mit der ersten Leistung zu bestimmen, und wenn die Schritte c-d mit einer zweiten Leistung wiederholt werden, das erste Vorerwärmungs-ERG-Signal und/oder das erste Nacherwärmungs-ERG-Signal mit dem zweiten Erwärmungs-ERG-Signal vergleicht, um den Temperaturanstieg des Netzhautgewebes mit der zweiten Leistung zu bestimmen, und wenn die Schritte c-d mit einer nachfolgenden Leistung wiederholt werden, das erste Vorerwärmungs-ERG-Signal und/oder das erste Nacherwärmungs-ERG-Signal mit dem nachfolgenden Erwärmungs-ERG-Signal zu vergleichen, um den Temperaturanstieg des Netzhautgewebes mit der nachfolgenden Erwärmungsleistung zu bestimmen
i. die ermittelte Temperaturerhöhung des Netzhautgewebes mit der ersten Leistung nutzt, um die Temperaturerhöhung des Zielbereichs pro Einheit der Erwärmungsleistung zu bestimmen, und wenn die Temperaturerhöhung des Netzhautgewebes mit einer zweiten Leistung ermittelt wird, die ermittelte Temperaturerhöhung des Netzhautgewebes mit der zweiten Leistung nutzt, um die Temperaturerhöhung des Zielbereichs pro Einheit der Erwärmungsleistung zu bestimmen, und wenn die Schritte c-d mit einer nachfolgenden Leistung wiederholt werden, die ermittelte Temperaturerhöhung des Netzhautgewebes mit der nachfolgenden Leistung zu verwenden, um den Temperaturanstieg des Zielbereichs pro Einheit der Erwärmungsleistung zu bestimmen.

4. Anordnung nach Anspruch 3, wobei der Prozessor so konfiguriert ist, dass er mindestens die Schritte c-d mit einem vorbestimmten Satz von Erwärmungsleistungen wiederholt und basierend auf der erhaltenen Vielzahl von Temperaturerhöhungen des Zielbereichs pro Einheit der Erwärmungsleistung als Indikator eine aggregierte Temperaturerhöhung des Zielbereichs pro Einheit der Erwärmungsleistung bestimmt.

5. Anordnung nach Anspruch 3 oder 4, wobei der Prozessor so konfiguriert ist, dass er ERG-Signale, die zwischen einem vorbestimmten Zeitpunkt nach dem Ändern oder Einleiten der Erwärmung der Netzhaut und einer anschließenden Beendigung oder Änderung der Erwärmung der Netzhaut erhalten werden, als Erwärmungs-ERG-Signale zur Temperaturbestimmung verwendet.

6. Anordnung nach Anspruch 3 oder 4, wobei der Prozessor so konfiguriert ist, dass er ERG-Signale, die im Wesentlichen unmittelbar nach Beginn der Erwärmung erhalten werden, als Erwärmungs-ERG-Signale zur Temperaturbestimmung verwendet, um eine durch die Erwärmung zu Beginn des Erwärmungsvorgangs verursachte Temperaturanstiegsrate im Zielbereich der Netzhaut zu bestimmen.

7. Anordnung nach einem der vorstehenden Ansprüche, wobei der Prozessor so konfiguriert ist, dass er ein Kalibrierungsprotokoll ausführt, um eine Erwärmungsleistung zu bestimmen, die erwartet wird, um die Temperatur mindestens des Zielbereichs auf eine Zieltemperatur oder einen Temperaturanstieg anzuheben, und der Prozessor so konfiguriert ist, dass er kontinuierlich einen Kinetikparameter des ERG-Signals während der Erwärmung der Netzhaut durch die Anordnung bestimmt, die die Erwärmungsleistung basierend auf dem Kalibrierungsprotokoll bereitstellt, und der Prozessor ferner so konfiguriert ist, dass er eine Änderung des Kinetikparameters des ERG-Signals während der Erwärmung der Netzhaut ermittelt und die Änderung des Kinetikparameters mit einer erwarteten Änderung des Kinetikparameters vergleicht, basierend auf einer ermittelten Zieltemperatur oder Temperaturerhöhung, wobei die Anordnung so konfiguriert ist, dass sie die Erwärmung beendet oder anpasst, wenn die ermittelte Änderung des kinetischen Parameters um mehr als einen Schwellenwert von der erwarteten Änderung des kinetischen Parameters abweicht, und/oder wobei die Anordnung so konfiguriert ist, dass sie einen Benutzer der Anordnung informiert, wenn die ermittelte Änderung des kinetischen Parameters um mehr als einen Schwellenwert von der erwarteten Änderung des kinetischen Parameters abweicht.

8. Anordnung nach Anspruch 7, wobei der Schwellenwert der Differenz zwischen der erwarteten und der beobachteten Änderung des kinetischen Parameters einer Temperaturänderung zwischen 2 °C und 8 °C, vorzugsweise zwischen 4 °C und 6 °C, entspricht.

9. Anordnung eines vorstehenden Anspruchs, wobei der Prozessor so konfiguriert ist, dass er eine Erwärmungsleistung extrapoliert, die einen vorbestimmten Temperaturanstieg im Zielbereich oder eine vorbestimmte absolute Temperatur im Zielbereich in Fällen bereitstellt, in denen eine Körpertemperatur bestimmt wird.

10. Anordnung nach Anspruch 5, wobei der Prozessor so konfiguriert ist, dass er extrapoliert, wie sich die ERG-Signalkinetik während der Behandlung mit höherer Leistung verändern sollte, und so konfiguriert ist, dass er die Erwärmung der Netzhaut beendet oder die Behandlungsleistung verringert, wenn die Veränderung der Kinetik um einen vorbestimmten Wert von der Erwartung abweicht.

11. Anordnung nach einem der vorstehenden Ansprüche, wobei der Prozessor so konfiguriert ist, dass er das Signal-Rausch-Verhältnis des ERG-Signals bestimmt und die Erwärmung der Netzhaut beendet, wenn das Signal-Rausch-Verhältnis unter einem vorgegebenen Schwellenwert liegt.

12. Anordnung nach einem der vorstehenden Ansprüche, wobei die relative Schwellenamplitude zwischen der während der Erwärmung der Netzhaut bestimmten Amplitude des ERG-Signals und der Amplitude einer zuvor bestimmten Referenz-ERG-Reaktionsamplitude zwischen 0,4 und 0,9, vorzugsweise zwischen 0,5 und 0,7 liegt.

13. Anordnung eines vorstehenden Anspruchs, wobei der Prozessor so konfiguriert ist, dass er den Schwellenwert der Änderung der Kinetik entsprechend einer Temperaturänderung zwischen 0,5 °C und 4 °C, vorteilhafterweise zwischen 1 °C und 2 °C, bestimmt, wobei der Prozessor so konfiguriert ist, dass er eine Beziehung zwischen Kinetik und Temperatur basierend auf einer zuvor bestimmten Änderung der Kinetik pro Grad Temperaturänderung in einem vorbestimmten therapeutischen Fenster der Netzhauttemperatur bestimmt.

14. Anordnung nach einem der vorstehenden Ansprüche, wobei der Prozessor so konfiguriert ist, dass er als Indikator die Impedanz zwischen den ERG-Elektroden basierend auf dem erhaltenen ERG-Signal bestimmt und die Einleitung der Erwärmung der Netzhaut beendet oder deaktiviert, wenn die Impedanz über einem vorbestimmten Schwellenwert liegt.

## Revendications

1. Dispositif d'électrorétinographie rétinienne, ERG, de stimulation et de chauffage, le dispositif comprenant au moins un processeur (102) et au moins une source lumineuse (LED3) fournissant au moins un faisceau de stimulation pour induire un signal ERG à partir d'une zone cible de la rétine, le dispositif comprenant également un système de chauffage pour élever la température au moins de la zone cible, dans lequel le processeur est configuré pour recevoir un signal ERG rétinien induit par le faisceau de stimulation pendant le chauffage rétinien, déterminer, sur la base dudit signal ERG, un ou plusieurs indicateurs indiquant une température de la rétine, et commander le système de chauffage sur la base dudit indicateur, ledit indicateur comprenant au moins l'amplitude du signal ERG et/ou la cinétique du signal ERG.
**caractérisé en ce que** :
le processeur est également configuré pour comparer l'amplitude à une amplitude de réponse ERG de référence préalablement déterminée, et pour interrompre ou ajuster le chauffage rétinien si l'amplitude du signal ERG pendant le chauffage rétinien tombe en dessous d'un seuil prédéterminé par rapport à l'amplitude de réponse ERG de référence préalablement déterminée et/ou pour informer un utilisateur du dispositif que l'amplitude du signal ERG pendant le chauffage rétinien tombe en dessous d'un seuil prédéterminé par rapport à l'amplitude de réponse ERG de référence préalablement déterminée,
et/ou **en ce que** le processeur est configuré pour comparer la cinétique du signal ERG à une cinétique préalablement déterminée, et pour interrompre ou ajuster le chauffage rétinien si la cinétique est plus lente que la cinétique préalablement déterminée de plus d'un seuil prédéterminé et/ou pour informer un utilisateur du dispositif que la cinétique est plus lente que la cinétique préalablement déterminée de plus d'un seuil prédéterminé.

2. Dispositif selon la revendication 1, dans lequel le dispositif est configuré pour exécuter un protocole d'étalonnage et déterminer, à titre d'indicateur, l'élévation de température par unité de puissance de chauffage pour la zone cible du fond d'œil, et utiliser ledit indicateur pour commander le système de chauffage afin de fournir une puissance de chauffage étalonnée pour élever la température de la rétine.

3. Dispositif selon la revendication 2, dans lequel le processeur est configuré pour :
a. initier une stimulation rétinienne en commandant l'au moins la source lumineuse pour fournir le faisceau de stimulation
b. déterminer un premier signal ERG de référence de préchauffage
c. initier un chauffage rétinien en commandant le système de chauffage afin de fournir un chauffage d'une zone cible avec une première puissance et poursuivre le chauffage rétinien pendant une durée prédéfinie
d. déterminer un premier signal ERG de chauffage
e. répéter éventuellement les étapes c à d avec une seconde puissance ou une puissance ultérieure pour déterminer les seconds signaux ERG de chauffage ou les signaux ERG de chauffage ultérieurs
f. interrompre le chauffage rétinien
g. déterminer éventuellement un premier signal ERG de référence après chauffage
h. comparer le premier signal ERG de préchauffage et/ou le premier signal ERG de post-chauffage au premier signal ERG de chauffage pour déterminer l'élévation de température du tissu rétinien avec la première puissance, et si les étapes c à d sont répétées avec une seconde puissance, comparer le premier signal ERG de préchauffage et/ou le premier signal ERG de post-chauffage au second signal ERG de chauffage pour déterminer l'élévation de température du tissu rétinien avec la seconde puissance, et si les étapes c à d sont répétées avec une puissance ultérieure, comparer le premier signal ERG de préchauffage et/ou le premier signal ERG de post-chauffage au signal ERG de chauffage ultérieur pour déterminer l'élévation de température du tissu rétinien avec la puissance de chauffage ultérieure
i. utiliser l'élévation de température déterminée du tissu rétinien avec la première puissance pour déterminer l'augmentation de température de la zone cible par unité de puissance de chauffage, et si l'élévation de température du tissu rétinien est déterminée avec une seconde puissance, utiliser l'élévation de température déterminée du tissu rétinien avec la seconde puissance pour déterminer l'augmentation de température de la zone cible par unité de puissance de chauffage, et si les étapes c à d sont répétées avec une puissance ultérieure, utiliser l'élévation de température déterminée du tissu rétinien avec la puissance ultérieure pour déterminer l'augmentation de température de la zone cible par unité de puissance de chauffage.

4. Dispositif selon la revendication 3, dans lequel le processeur est configuré pour répéter au moins les étapes c à d avec un ensemble prédéterminé de puissances de chauffage et, sur la base de la pluralité obtenue d'augmentations de température de la zone cible par unité de puissance de chauffage, déterminer, à titre d'indicateur, une augmentation de température globale de la zone cible par unité de puissance de chauffage.

5. Dispositif selon la revendication 3 ou 4, dans lequel le processeur est configuré pour utiliser des signaux ERG obtenus entre un temps prédéterminé après la variation ou le démarrage du chauffage rétinien et une fin ou une variation ultérieure du chauffage rétinien comme signaux ERG de chauffage pour la détermination de la température.

6. Dispositif selon la revendication 3 ou 4, dans lequel le processeur est configuré pour utiliser des signaux ERG obtenus essentiellement immédiatement après le début du chauffage comme signaux ERG de chauffage pour la détermination de la température afin de déterminer un taux d'augmentation de température dans la zone cible de la rétine causée par le chauffage au début de la procédure de chauffage.

7. Dispositif selon une quelconque revendication précédente, dans lequel le processeur est configuré pour exécuter un protocole d'étalonnage afin de déterminer une puissance de chauffage censée élever la température d'au moins la zone cible à une température cible ou à une élévation de température, et le processeur est configuré pour déterminer en continu un paramètre cinétique du signal ERG pendant le chauffage rétinien par le dispositif fournissant la puissance de chauffage sur la base dudit protocole d'étalonnage, et le processeur est également configuré pour déterminer une variation du paramètre cinétique du signal ERG pendant le chauffage rétinien et comparer la variation du paramètre cinétique à une variation attendue du paramètre cinétique sur la base d'une température cible ou d'une élévation de température déterminée, dans lequel le dispositif est configuré pour interrompre ou ajuster le chauffage si la variation du paramètre cinétique mesurée diffère de la variation attendue de plus d'une quantité seuil, et/ou dans lequel le dispositif est configuré pour informer un utilisateur du dispositif si la variation déterminée du paramètre cinétique diffère de la variation attendue du paramètre cinétique de plus d'une quantité seuil.

8. Dispositif selon la revendication 7, dans lequel la quantité seuil de la différence entre la variation attendue et observée du paramètre cinétique correspond à une variation de température comprise entre 2 °C et 8 °C, avantageusement entre 4 °C et 6 °C.

9. Dispositif selon une quelconque revendication précédente, dans lequel le processeur est configuré pour extrapoler une puissance de chauffage qui fournit une élévation de température prédéterminée dans la zone cible ou une température absolue prédéterminée dans la zone cible, dans des cas où la température corporelle est déterminée.

10. Dispositif selon la revendication 5, dans lequel le processeur est configuré pour extrapoler comment la cinétique de signalisation ERG devrait changer pendant le traitement à une puissance plus élevée et est configuré pour interrompre le chauffage rétinien ou réduire la puissance de traitement si la variation de cinétique s'écarte de l'attente de plus d'une quantité prédéterminée.

11. Dispositif selon une quelconque revendication précédente, dans lequel le processeur est configuré pour déterminer le rapport signal/bruit du signal ERG et interrompre le chauffage rétinien si le rapport signal/bruit est inférieur à une valeur seuil prédéterminée.

12. Dispositif selon une quelconque revendication précédente, dans lequel l'amplitude relative seuil entre l'amplitude du signal ERG déterminée pendant le chauffage rétinien et l'amplitude d'une amplitude de réponse ERG de référence déterminée préalablement est comprise entre 0,4 et 0,9, avantageusement entre 0,5 et 0,7.

13. Dispositif selon une quelconque revendication précédente, dans lequel le processeur est configuré pour déterminer la quantité seuil de variation de la cinétique correspondant à une variation de température comprise entre 0,5 °C et 4 °C, avantageusement entre 1 °C et 2 °C, dans lequel le processeur est configuré pour déterminer une relation entre la cinétique et la température sur la base d'une variation de cinétique préalablement déterminée par degré de variation de température dans une fenêtre thérapeutique prédéterminée de température rétinienne.

14. Dispositif selon une quelconque revendication précédente, dans lequel le processeur est configuré pour déterminer, à titre d'indicateur, l'impédance entre des électrodes ERG sur la base dudit signal ERG obtenu et pour interrompre ou désactiver le déclenchement du chauffage rétinien si l'impédance est supérieure à une valeur seuil prédéterminée.
